(19) Europäisches Patentamt / European Patent Office / Office européen des brevets

(11) EP 3 394 176 B1

(12) # EUROPÄISCHE PATENTSCHRIFT

(45) Veröffentlichungstag und Bekanntmachung des Hinweises auf die Patenterteilung:
**24.06.2020 Patentblatt 2020/26**

(21) Anmeldenummer: **16801764.8**

(22) Anmeldetag: **25.11.2016**

(51) Int Cl.:
***C08L 83/04*** *(2006.01)* ***A61K 8/891*** *(2006.01)*

(86) Internationale Anmeldenummer:
**PCT/EP2016/078819**

(87) Internationale Veröffentlichungsnummer:
**WO 2017/108332 (29.06.2017 Gazette 2017/26)**

(54) **SILOXANELASTOMERE MIT SOFORTIGER OPTISCH UND SENSORISCH GLÄTTENDER WIRKUNG SOWIE SOLCHE SILIKONELASTOMERE ENTHALTENDE TOPISCHE HAUTPFLEGE-ZUSAMMENSETZUNGEN**

SILOXANE ELASTOMERS HAVING AN IMMEDIATE OPTICAL AND SENSORIAL SMOOTHING EFFECT AND TOPICAL SKIN-CARE COMPOSITIONS CONTAINING SUCH SILICONE ELASTOMERS

ÉLASTOMÈRES SILOXANE À ACTION LISSANTE INSTANTANÉE À LA VUE ET AU TOUCHER ET COMPOSITIONS DE SOIN POUR LA PEAU CONTENANT LESDITS ÉLASTOMÈRES SILOXANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **21.12.2015 DE 102015226221**

(43) Veröffentlichungstag der Anmeldung:
**31.10.2018 Patentblatt 2018/44**

(73) Patentinhaber: **Beiersdorf AG**
**20253 Hamburg (DE)**

(72) Erfinder:
- **PRUNS, Julia**
  **20144 Hamburg (DE)**
- **PESCHKE, Lisa**
  **97616 Bad Neustadt (DE)**
- **RASCHKE, Thomas**
  **25421 Pinneberg (DE)**
- **KUHLMANN, Imke**
  **22305 Hamburg (DE)**

(56) Entgegenhaltungen:
**WO-A1-2014/105970 WO-A1-2014/188667**
**DE-A1- 10 155 792**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 394 176 B1

## Beschreibung

Die Erfindung betrifft kosmetische oder dermatologische Zubereitungen enthaltend ein oder mehrere Silikonelastomere gemäß dem Wortlaut von Anspruch 1.

Viele Körperpflegeprodukte, die derzeit für den Verbraucher erhältlich sind, sind in erster Linie darauf gerichtet, die Gesundheit oder das äußere Erscheinungsbild der Haut oder des Haars zu verbessern. Von den Hautpflegeprodukten sind viele darauf gerichtet, die Faltenbildung der Haut und andere histologische Veränderungen, die in der Regel mit der Hautalterung oder mit einer Schädigung der Haut durch Umwelteinflüsse in Zusammenhang stehen, zu verzögern, zu minimieren oder sogar zu eliminieren.

Es wurden in der Technik zahlreiche Verbindungen als geeignet für die Regulierung des Hautzustands, einschließlich der Regulierung feiner Linien, Falten und anderer Formen von ungleichmäßiger oder rauer Oberflächenbeschaffenheit im Zusammenhang mit gealterter oder lichtgeschädigter Haut beschrieben.

Die Haut ist Belastungen durch zahlreiche äußere und innere Faktoren ausgesetzt. Extrinsische Faktoren umfassen Ultraviolettstrahlung (z. B. Sonnenbestrahlung), Umweltverschmutzung, Wind, Hitze, niedrige Feuchtigkeit, aggressive Tenside, Schleifmittel und dergleichen.

Intrinsische Faktoren umfassen chronologisches Alter und andere biochemische Veränderungen von innerhalb der Haut. Ob nun äußerlich oder innerlich, diese Faktoren resultieren in sichtbaren Zeichen der Hautalterung und der Schädigung durch Umwelteinflüsse, wie der Entstehung von Falten und anderen Formen der Rauigkeit (einschließlich vergrößerter Poren, Schuppenbildung und Hautlinien) sowie anderen histologischen Veränderungen im Zusammenhang mit Hautalterung oder -schädigung. Hautfalten erinnern viele Menschen an das Verschwinden der Jugend.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:

a) Trockenheit, Rauigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomenen.

Hautunebenheiten, beispielsweise kleine Fältchen, sind besonders unerwünschte Begleiterscheinungen der Hautalterung. Eines der Ziele kosmetischer Mittel ist es, diese Erscheinung entweder zu beseitigen, zu überspielen und/oder zu minimeren. Ein Weg besteht darin, der Haut Pflegekomponenten oder Wirkstoffe zuzufügen, was aber ein langwieriges Unterfangen darstellen kann.

Produkte zur Pflege gealterter Haut enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft Hautreizungen. Retinoide sind daher nur in geringen Konzentrationen einsetzbar.

Erwünscht ist dabei ein sofort nach der Anwendung des kosmetischen Mittels sichtbarer Effekt, wobei aber andererseits verhindert werden soll, dass eine "maskenhafte" Anmutung des betroffenen Hautareals bewirkt wird.

Es ist bekannt, dass durch die Verwendung sogenannter "Soft-Focus-Rohstoffe" kleinere Hautunebenheiten und Fältchen optisch kaschiert werden können. Solche Rohstoffe zeichnen sich durch eine geeignete Lichtbrechung und Lichtstreuung aus und mildern durch ihr diffuses Streuverhalten die Oberflächenerscheinungen der Haut.

Siloxanelastomere werden in kosmetischen Zubereitungen aufgrund ihrer seidigen und pudrigen Sensorikeigenschaften eingesetzt. Siloxanelastomere sind dreidimensional vernetzte Silikonpolymere. Im Unterschied zu den nicht vernetzten Silikonpolymeren weisen sie andere Eigenschaften als diese auf. Obwohl sie oftmals über 80 % an eingeschlossenem Silikonöl enthalten, verursachen sie meistens kein öliges, sondern im Gegenteil ein samtiges bis pudriges Hautgefühl. Auch Silkonelastomere sind als "Soft-Focus-Rohstoffe" vorgeschlagen worden.

In der US 4,256,870 werden vernetzte Siloxanpolymere unter Verwendung eines Vinyl- und eines Hydrosilans beschrieben.

In der EP 381 166 und der EP 383 540 werden die Grundlage der Reaktion aus einem Hydrosilan und einem Vinylsilan unter Platinkatalyse sowie die Verwendung der erhaltenen Silioxanelastomere in kosmetischen Zubereitungen beschrieben.

DE10155792 beschreibt additionsvernetzbare Zusammensetzungen auf Basis von Siloxanelastomeren für kosmetische Anwendung.

Eine Aufgabe der vorliegenden Erfindung war es, kosmetische Zubereitungen zur Verfügung zu stellen, die einerseits breite Einsatzmöglichkeiten bieten, andererseits auch nach Anwendung ein möglichst natürlich anmutendes Erscheinungsbild der Haut gewährleisten und gleichzeitig die Anwesenheit von Hautunebenheiten, insbesondere kleiner Fältchen, zu vermindern oder sogar gänzlich zu kaschieren.

Gelöst wird diese Aufgabe durch Zubereitungen auf Basis von Siloxanelastomeren, erhältlich, indem die Reaktion von

**A** einem oder mehreren oligomeren Organohydrogensiloxanen mit mindestens zwei SiH tragenden Siloxaneinheiten pro Molekül der folgenden Struktur

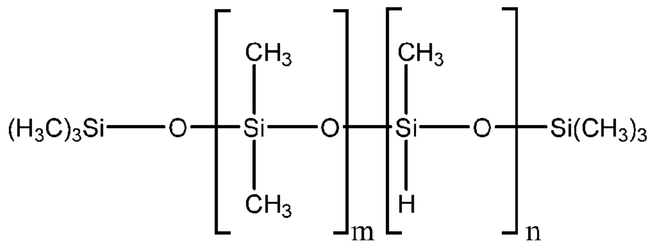

wobei m = 0 - 40, bevorzugt 0 - 30, insbesondere bevorzugt 0 - 20, und n=2 - 25 bevorzugt 2 - 15 , insbesondere bevorzugt 4-10

und

**B** einem oder mehreren oligomeren Divinylsiloxanen der folgenden Struktur

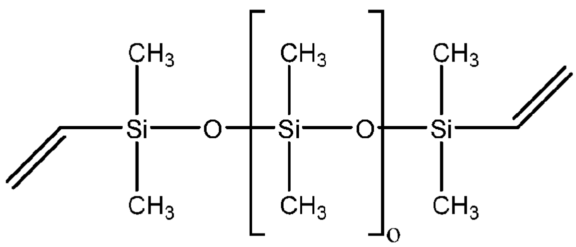

mit O = 5 - 90, bevorzugt 10 - 80 und besonders bevorzugt 15 - 70,

unter Verwendung von

**C** Platin(0)-1,2-divinyl-1,1,3,3-tetramethyldisiloxan, welches durch die Struktur

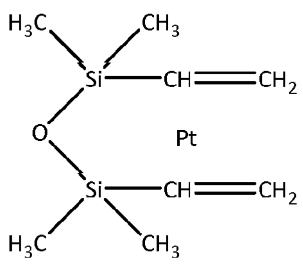

gekennzeichnet ist, als Hydrosilylationskatalysator, durchgeführt wird, wobei das Divinylsilan in einem Silikonöl (vorteilhaft Dimethicon) gelöst und unter Rühren auf Temperaturen im Bereich von 25 - 45 °C erwärmt wird, und wobei nach Einrühren des Katalysators das Organohydrogensiloxan zugegeben wird.

Die Reaktion startet unverzüglich und ist abgeschlossen, sobald sich die Konsistenz im gesamten Reaktionsgefäß erhöht hat und nicht mehr weiter erhöht.

Anschließend wird das Polymer unter Zugabe von weiterem Lösungsmedium kontrolliert geschert. Dies wird mit Hilfe eines Rotor-Stator Rührsystems (z.B. "L5M-A" der Firma Silverson) durchgeführt.

Hierfür wird eine Menge des vernetzten Silikonpolymers mit einer zusätzlichen Menge an Silikonöl (z.B Dimethicon) in ein geeignetes Gefäß gegeben und mit Hilfe des Rührsystems für eine definierte Zeit geschert. Diese Zeit ist sehr stark davon abhängig, wie groß der zu scherende Ansatz ist. Zum einen muss die Zeit ausreichen, um das Vernetzungsprodukt komplett zu scheren (und nicht nur einen Teil davon). Zum anderen darf die Zeit nicht zu lang gewählt werden, da ein Zuviel an Scherung unter Umständen das Polymer zerstört und die dreidimensionale Vernetzung einschänkt. Die Einwaagen an Polymer und Silikonöl werden dabei so gewählt, dass eine Polymerkonzentration von 5 bis 20% erreicht wird. Als besonders vorteilhaft hat sich eine Polymerkonzentration von 15 - 20% ergeben.

Für einen Ansatz von 1 kg kann z.B. für eine Minute mit Hilfe des Hochschermischers "L5M-A" der Firma Silverson (unter Verwendung des "Vielzweckzerkleinerungsstators" und einer Umdrehungszahl von 8000rpm) eine geeignete Scherung erreicht werden. Wichtig ist, auf eine gleichmäßige Scherung des gesamten Produktes zu achten. Je nach Größe des Reaktionssystems kann die Dauer der Scherung erheblich variieren von einer Minute bis zu 15 min und mehr.

Die erfindungsgemäße kosmetische Formulierung umfasst auch kosmetische Zubereitungen mit einem Gehalt an solchen Silikonelastomeren.

Ein Verfahren zur Herstellung von Silikonelastomeren wird beschrieben, das dadurch gekennzeichnet ist, dass

**A** einem oder mehreren oligomeren Organohydrogensiloxanen mit mindestens zwei SiH tragenden Siloxaneinheiten pro Molekül der folgenden Struktur

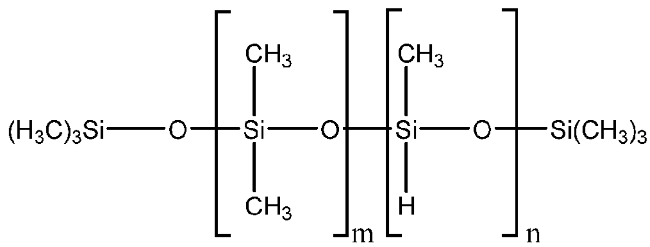

wobei m = 0 - 40, bevorzugt 0 - 30, insbesondere bevorzugt 0 - 20, und n=2 - 25 bevorzugt 2 - 15 , insbesondere bevorzugt 4-10

und

**B** einem oder mehreren oligomeren Divinylsiloxanen der folgenden Struktur

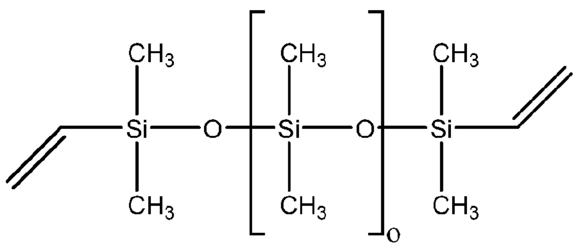

mit o = 5 - 90, bevorzugt 10 - 80 und besonders bevorzugt 15 - 70,

unter Verwendung von

**C** Platin(0)-1,2-divinyl-1,1,3,3-tetramethyldisiloxan, welches durch die Struktur

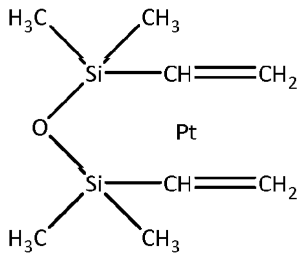

gekennzeichnet ist, als Hydrosilylationskatalysator, durchgeführt wird, wobei das Divinylsilan in einem Silikonöl (vorteilhaft Dimethicon) gelöst und unter Rühren auf Temperaturen im Bereich von 20 - 60 °C, bevorzugt 25 - 45 °C erwärmt wird, und wobei nach Einrühren des Katalysators das Organohydrogensiloxan zugegeben wird.

Vorteilhaft werden molare Verhältnisse von A zu B aus dem Bereich von 10:1 bis 1:10, bevorzugt von 5:1 bis 1:5, insbesondere bevorzugt von 2:1 bis 1:2 gewählt.

Nach Einrühren des Katalysators werden die Organohydrogensiloxane zugegeben. Die Reaktion startet unverzüglich und ist abgeschlossen, sobald sich die Konsistenz im gesamten Reaktionsgefäss erhöht hat.

Anschließend wird das Polymer unter Zugabe von weiterem Lösungsmedium kontrolliert geschert. Dies wird mit Hilfe eines Rotor-Stator Rührsystems (z.B. "L5M-A" der Firma Silverson) durchgeführt.

Hierfür wird eine Menge des vernetzten Silikonpolymers mit einer zusätzlichen Menge an Silikonöl (z.B Dimethicon) in ein geeignetes Gefäß gegeben und mit Hilfe des Rührsystems für eine definierte Zeit geschert. Diese Zeit ist sehr stark davon abhängig, wie groß der zu scherende Ansatz ist. Zum einen muss die Zeit ausreichen, um das Vernetzungsprodukt komplett zu scheren (und nicht nur einen Teil davon). Zum anderen darf die Zeit nicht zu lang gewählt werden, da ein Zuviel an Scherung unter Umständen das Polymer zerstört und die dreidimensionale Vernetzung einschänkt. Die Einwaagen an Polymer und Silikonöl werden dabei so gewählt, dass eine Polymerkonzentration von 5 bis 20% erreicht wird. Als besonders vorteilhaft hat sich eine Polymerkonzentration von 15 - 20% ergeben.

Für einen Ansatz von 1 kg kann z.B. für eine Minute mit Hilfe des Hochschermischers "L5M-A" der Firma Silverson (unter Verwendung des "Vielzweckzerkleinerungsstators" und einer Umdrehungszahl von 8000rpm) eine geeignete Scherung erreicht werden. Wichtig ist, auf eine gleichmäßige Scherung des gesamten Produktes zu achten. Je nach Größe des Reaktionssystems kann die Dauer der Scherung erheblich variieren von einer Minute bis zu 15 Min. und mehr.

Die erfindungsgemäßen Silikonelastomere zeichnen sich durch einen ausgeprägten "Soft-Focus-Effekt" aus, durch den kleinere Hautunebenheiten und Fältchen optisch kaschiert werden, was im Folgenden belegt werden soll.

Für einen Nachweis des Soft-Focus Effekts wird eine Messung der totalen und gestreuten Transmission, sowie der totalen und gestreuten Reflektion durchgeführt (mittels Photometer mit Ulbricht-Kugel). Nach Emmert (Cosm. Toil., 111, 1996, S.57-61) wird der Soft-Focus Effekt am besten erreicht, wenn eine möchlichst hoher Anteil an diffuser (=gestreuter) Transmission und eine hohe Gesamt-Transmission erreicht wird.

Die hohe Transmission bedeutet gleichzeitig eine geringe direkte Reflektion, damit Glanz der Haut vermieden wird und der natürliche Hautton durchscheint.

Die erfindungsgemäße Zusammensetzung und zu vergleichende Rohstoffe wurden als 30μm dicker Film auf einem Objektträger ausgestrichen und nach 3 Minuten Trocknungszeit mittels Zweistrahlphotometer (SPECORD 250 UV/VIS Spektralphotometer) unter Verwendung einer Ulbricht-Kugel (Analytik Jena AG) gemessen.

Der Anteil der diffusen Transmission wird insbesondere hoch bewertet, da hierdurch HautUnebenheiten kaschiert werden und der eigene Hautton durchscheint. Die hier gezeigten Werte sind Mittelwerte über den gesamten Wellenlängenbereich des sichtbaren Lichtes (400-800 nm). Siehe Fig. 1.

Als Vergleichsmuster wurden die folgenden gemessen:

1. DC 9041 (Dow Corning)
2. Silsoft Silicone Gel (Momentive)
3. KSG-19 (Shin Etsu)
4. CXG-1104 (Nusil)
5. Vaseline (Sasol Wax, Negativ-vergleich)
6. Erfindungsgemäße Zusammensetzung

Es zeigt sich, dass die erfindungsgemäße Zusammensetzung hier den höchsten Anteil an diffuser Transmission aufweist.

Weiterhin haben erfindungsgemäße Siloxanelastomere besonders klebrigkeitsmindernde Wirkung auf der

Haut. Die Minderung der Klebrigkeit wurde anhand von Formulierungen mit hohen Mengen an Lichtschutzfiltern, insbesondere wasserlöslichen, durchgeführt. Die Klebrigkeit dieser Formulierungen wurde mit Hilfe einer Messung der "in-vitro Sandanhaftung" durchgeführt.

In-Vitro Sandanhaftung:

Je 50 mg der zu testenden Emulsionen wurden auf PMMA Schönberg Platten (5,0 x 5,0 cm) aufgetragen und möglichst gleichmäßig mit einem Fingerling auf der Platte verteilt. Anschließend wird die aufgetragene Beispielrezeptur für 15 min. bei Raumtemperatur getrocknet. Danach wurde das Gewicht der getrockneten Platten mit einer Analysenwaage ermittelt. Anschließend wurden die Platten mit feinem Seesand (reinst, Merck KGaA, Darmstadt), übergossen. Durch einmaliges Rutschen der Platten auf einer dafür vorgesehenen Rutschvorrichtung wurde lose anhaftender Sand entfernt.

Der daraufhin auf der Platte zurückbleibende anhaftende Sand wurde durch Auswiegen ermittelt. Die Sandanhaftung kann mit folgender Gleichung ermittelt werden:

$$\Delta(\text{Anhaftung})\ [\text{mg}] = m(\text{Platte mit Sand})\ [\text{mg}] - m\ (\text{eingecremte Platte})\ [\text{mg}]$$

Der Versuch wurde je Beispielrezeptur 10-fach ausgeführt und die Ergebnisse gemittelt.

| | |
|---|---|
| Beispielrezeptur 6: | Rezeptur ohne Zusatz von Silikonen |
| Beispielrezeptur 7: | Rezeptur mit Zusatz von 10% Dimethicon |
| Beispielrezeptur 8: | Rezeptur mit Zusatz von 10% Dimethicon / Dimethicon Crosspolymer (DC 9041, Dow Corning) |
| Beispielrezeptur 9: | Rezeptur mit Zusatz von 10% Dimethicon / Vinyl Dimethicon Crosspolymer (KSG-19, Shin-Etsu) |
| Beispielrezeptur 10: | Rezeptur mit Zusatz von 10% Dimethicon / Vinyl Dimethicon Crosspolymer (erfindungsgemäße Zusammensetzung). |

Die genauen Zusammensetzungen sind in Tabelle 4 aufgeführt.

In Fig. 2 sind die Ergebnisse der Messungen zusammengefasst. Mit der erfindungsgemäßen Zusammensetzung (Bsp. 10) konnte die Menge an anhaftendem Sand (und damit der Klebrigkeit) im Vergleich zu den anderen Rezepturen gesenkt werden.

Bevorzugt enthalten kosmetische oder dermatologische Zubereitungen gemäß der Erfindung 0,1 - 60 Gew.-%, bevorzugt 1 - 51 Gew.-%, besonders bevorzugt 3 - 40 Gew.-% an einem oder mehreren der erfindungsgemäßen Silikonelastomere, bezogen auf die Gesamtzusammensetzung der Zubereitungen.

Es ist auch gegebenenfalls vorteilhaft, kosmetischen oder dermatologischen Zubereitungen gemäß der Erfindung 0,1 - 50 Gew.-%, besonders bevorzugt 1 - 30 Gew.-% an einem oder mehreren Silikonelastomeren anderer Herkunft als der Erfindung, bezogen auf die Gesamtzusammensetzung der Zubereitungen, zuzufügen.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:

- Wasser oder wässrige Lösungen
- Öle, wie Triglyceride der Caprin- oder der Caprylsäure
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

- Alkohole, Diole oder Polyole niedriger C-Zahl, sowie deren Ether, vorzugsweise Ethanol, Isopropanol, Propylenglykol, Glycerin, Hexandiol, Octandiol, Ethylhexylglycerin, Glycerylcaprylat, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft.

Die wässrige Phase der erfindungsgemäßen Zubereitungen enthält gegebenenfalls vorteilhaft ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Celluloseether wie z.B. Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, Ultrez 10, ETD 2020, jeweils einzeln oder in Kombination.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Rizinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1: Herstellung Elastomer mit besonders starken optischen Effekten**

Die Herstellung des Siloxan-Elastomers mit besonders starken optischen Effekten geschieht wie im Folgenden beschrieben:

**A** Zwei Organohydrogensiloxane (z.B. "Silmer H D0" (genannt "H1") und "Silmer H J2" (genannt "H2") (Siltech LLC, Lawrenceville, GA, U.S.A.)) werden mit

**B** Zwei Divinylsilanen (z.B. "Silmer Vin 15" (genannt "V1") und "Silmer Vin 70" (genannt "V2) (Siltech LLC, Lawrenceville, GA, U.S.A.)) unter Hilfe des Katalysators

**C** Platin(0)-1,2-divinyl-1,1,3,3-tetramethyldisiloxan (z.B. als Lösung in Polydimethylsiloxan (Sigma-Aldrich GmbH, München, Deutschland)

wie folgt in Dimethicon als Lösemittel zur Reaktion gebracht. Die Mengen der Ausgangsstoffe werden dabei so gewählt, dass die molare Menge der Silangruppen der Menge der Vinylgruppen entspricht. Folgende Mengen werden zur Reaktion gebracht:

**Tabelle 1. Zusammensetzung des Elastomers "Beispiel 1"**

| *Komponente* | *Menge (Gewichts-%)* |
|---|---|
| Organohydrogensiloxan "H1" | 0,6 |
| Organohydrogensiloxan "H2" | 0,85 |
| Divinylsilan "V1" | 2,1 |
| Divinylsilan "V2" | 16,44 |
| Platin(0)-1,2-divinyl-1,1,3,3-tetramethyldisiloxan, 0.1 M in Polydimethysiloxan | 2 ppm (=0,015% d. Lösung) |
| Dimethicon 5 cSt | 80 |

Die Vinyl-Komponenten V1 und V2 werden in Dimethicon eingerührt und auf 40°C erwärmt. Wenn die Temperatur erreicht ist, wird die Katalysatorlösung hinzugegeben. Ist diese homogen verteilt, so werden die Organohydrogensiloxane H1 und H2 hinzugegeben. Die Lösung wird weiterhin gerührt, die Reaktion startet unverzüglich. Das Ende des Polymerisationsprozesses zeigt sich anhand einer Gelierung der Reaktionsmasse und einem damit verbundenen rapiden Viskositätsanstieg. Wenn sich die Viskosität der Produktmasse nicht mehr wesentlich erhöht, ist die Reaktion abgeschlossen. Das kann je nach Volumen der Reaktionsmasse unterschiedlich lange dauern. Die Polymerkonzentration in dem fertigen Reaktionsprodukt beträgt 20%.

Zur Fertigstellung des Elastomerproduktes "Beispiel 1" wird dieses noch unter Zugabe von weiterem Dimethicon (5 cSt) und starker Scherung auf eine Polymerkonzentration von 17.5% gebracht. Hierfür wird eine entsprechende Menge an Reaktionsprodukt in ein geeignetes Rührgefäß eingewogen und - noch ohne Zugabe von weiterem Dimethicon - für 40 Sekunden mit Hilfe des Hochschermischers "L5M-A" der Firma Silverson (unter Verwendung des "Vielzweckzerkleinerungsstators" und einer Umdrehungszahl von 8000rpm) geschert.

Anschließend gibt man eine entsprechende Menge Dimethicon 5 cSt hinzu und arbeitet dieses mit dem Mischer ein. Wichtig ist, auf eine gleichmäßige Scherung des gesamten Produktes zu achten.

**Beispiel 2: Herstellung Elastomer mit besonders klebrigkeitsmindernden Effekten**

Die Herstellung des Siloxan-Elastomers mit besonders starken sensorischen Effekten geschieht wie im Folgenden beschrieben:

**A** Ein Organohydrogensiloxan (z.B. "Silmer H D5" (genannt "H1", Siltech LLC, Lawrenceville, GA, U.S.A.)) wird mit

**B** einem Divinylsilan (z.B. "Silmer Vin D5" (genannt "V1", Siltech LLC, Lawrenceville, GA, U.S.A.)) unter Hilfe des Katalysators

**C** Platin(0)-1,2-divinyl-1,1,3,3-tetramethyldisiloxan (z.B. als Lösung in Polydimethylsiloxan (Sigma-Aldrich GmbH, München, Deutschland)

wie folgt mit Dimethicon als Lösemittel zur Reaktion gebracht:
Die Mengen der Ausgangsstoffe werden dabei so gewählt, dass die molare Menge der Silangruppen der Menge der Vinylgruppen entspricht. Folgende Mengen werden zur Reaktion gebracht:

**Tabelle 2. Zusammensetzung des Elastomers "Beispiel 2"**

| *Komponente* | *Menge (Gewichts-%)* |
|---|---|
| Organohydrogensiloxan "H1" | 1,84 |
| Divinylsilan "V1" | 18,15 |
| Platin(0)-1,2-divinyl-1,1,3,3-tetramethyldisiloxan, 0.1 M in Polydimethysiloxan | 2 ppm (=0,015% d. Lösung) |
| Dimethicon 5 cSt | 80 |

Die Vinyl-Komponente V1 wird in Dimethicon eingerührt und auf 40°C erwärmt. Wenn die Temperatur erreicht ist, wird die Katalysatorlösung hinzugegeben. Ist diese homogen verteilt, so wird das Organohydrogensiloxan H1 hinzugegeben. Die Lösung wird weiterhin gerührt, die Reaktion startet unverzüglich. Das Ende des Polymerisationsprozesses zeigt sich anhand einer Gelierung der Reaktionsmasse und einem damit verbundenen rapiden Viskositätsanstieg. Die Polymerkonzentration in dem fertigen Reaktionsprodukt beträgt 20%.

Zur Fertigstellung des Elastomerproduktes "Beispiel 2" wird dieses noch unter Zugabe von weiteren Dimethicon (5 cSt) und starker Scherung auf eine Polymerkonzentration von 17.5% gebracht. Hierfür wird eine entsprechende Menge an Reaktionsprodukt in ein geeignetes Rührgefäß eingewogen, mit einer entsprechenden Menge Dimethicon 5 cSt versetzt für 120 Sekunden mit Hilfe des Hochschermischers "L5M-A" der Firma Silverson (unter Verwendung des "Vielzweckzerkleinerungsstators" und einer Umdrehungszahl von 8000rpm) geschert.

**Tabelle 3.**

| Beispiele 3-5: Emulsionen mit einem Gehalt an erfindungsgemäßen Siloxanelastomeren | | | |
|---|---|---|---|
| **Beispiel Nummer** | **3** | **4** | **5** |
| PG-10 Stearat | | 0,5 | |
| Bis-(Glyceryl/Lauryl) Glyceryl Lauryl Dimethicone (and) Caprylic/Capric Triglyceride (Abil EM 120) | | | 3 |
| Natrium Stearoylglutamat | 0,2 | | |
| Cetylstearylalkohol | 1 | 1 | |
| Caprylic/Capric Triglyceride | 1 | 5 | |
| Octyldodecanol | 5 | 5 | |
| Dimethicone | 8 | 5 | 7 |
| Ethylhexylstearat | 3 | | 3 |
| Ethylhexylmethoxycinnamat | | 2 | |
| 2-Ethylhexyl-2-cyano-3-diphenylacrylat (Octocrylen) | | 2 | |
| Ethylhexyltriazon | | 0,5 | |
| Butylmethoxydibenzoylmethan | | 1 | |
| Bis-Ethylhexyloxyphenol-methoxyphenyltriazine | | 1 | |
| Ubichinon (Q10) | | | 0,1 |
| Tocopherylacetat | | | 0,5 |
| Methylpropandiol | 3 | | 2 |
| Trinatrium EDTA | | 0,1 | |
| Glycerin | 5 | 10 | 3 |
| Panthenol | 0,5 | | |
| Phenoxyethanol | 0,5 | 0,5 | 0,3 |
| Ethanol denaturiert | 1 | 3 | |
| Xanthan Gummi | 0,2 | | |
| Polyacrylsäure (Carbomer) | 0,2 | 0,2 | |
| C10-30 Alkylacrylat crosspolymer | 0,2 | 0,2 | |
| *Silicon-Elastomer gemäß Beispiel 1* | 50 | | 40 |
| *Silicon-Elastomer gemäß Beispiel 2* | | 25 | |
| Füllstoffe/Additive (Distärkephosphat, Talkum, Bentonit, Silica, Eisenoxide) | 2 | 1 | 1 |
| Parfüm | q.s. | q.s. | q.s. |
| Wasser | Ad 100 | Ad 100 | Ad 100 |

**Tabelle 4.**

| Zusammensetzungen der Beispielformulierungen für die Ermittlung der "In-Vitro Sandanhaftung" | | | | | |
|---|---|---|---|---|---|
| **INCI** | **Bsp. 6** | **Bsp. 7** | **Bsp. 8** | **Bsp. 9** | **Bsp. 10** |
| Dimethicon | | 10 | | | |
| Dimethicon + Dimethicon Crosspolymer (DC 9041, Dow Corning) | | | 10 | | |
| Dimethicon + Dimethicon/Vinyl Dimethicon Crosspolymer (KSG-19, Shin-Etsu) | | | | 10 | |
| Silikon-Elastomer (erfindungsgemäße Zusammensetzung gem. Beispiel 1) | | | | | 10 |
| Natrium Stearoylglutamat | 0.3 | 0.3 | 0.3 | 0.3 | 0.3 |
| C12-15 Alkylbenzoat | 2 | 2 | 2 | 2 | 2 |
| Glycerin + Wasser | 7 | 7 | 7 | 7 | 7 |
| Methylpropandiol | | | 0.4 | 0.4 | 0.4 |
| Wasser + Natronlauge | 1.54 | 1.54 | 1.54 | 1.54 | 1.54 |
| Phenoxyethanol | 0.6 | 0.6 | 0.6 | 0.6 | 0.6 |
| Cetylstearylalkohol | 2 | 2 | 2 | 2 | 2 |
| Ammonium Acryloyldimethyltaurat/VP | 0.4 | 0.4 | 0.4 | 0.4 | 0.4 |
| Copolymer + Wasser | | | | | |
| Acrylat/C10-30 Alkyl Acrylat Crosspolymer | 0.25 | 0.25 | 0.25 | 0.25 | 0.25 |
| Xanthangummi | 0.1 | 0.1 | 0.1 | 0.1 | 0.1 |
| Wasser | 67.5 | 57.5 | 57.5 | 57.5 | 57.5 |
| Alkohol + Wasser | 0.4 | 0.4 | 0.4 | 0.4 | 0.4 |
| Wasser + Trinatrium EDTA | 1 | 1 | 1 | 1 | 1 |
| Octocrylen | 8 | 8 | 8 | 8 | 8 |
| Butyl Methoxydibenzoylmethan | 4.5 | 4.5 | 4.5 | 4.5 | 4.5 |
| Phenylbenzimidazol-Sulfönsäure | 4 | 4 | 4 | 4 | 4 |

## Patentansprüche

**1.** Kosmetische oder dermatologische Zubereitungen, enthaltend ein oder mehrere Silikonelastomere, welches erhältlich ist oder welche erhältlich sind, indem die Reaktion von

**A** einem oder mehreren oligomeren Organohydrogensiloxanen mit mindestens zwei SiH tragenden Siloxaneinheiten pro Molekül der folgenden Struktur

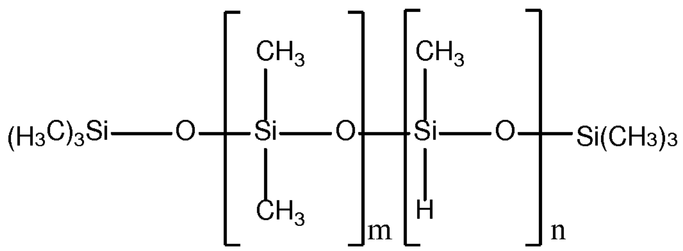

wobei m = 0 - 40, bevorzugt 0 - 30, insbesondere bevorzugt 0 - 20, und n=2 - 25 bevorzugt 2 - 15 , insbesondere bevorzugt 4-10
und
B einem oder mehreren oligomeren Divinylsiloxanen der folgenden Struktur

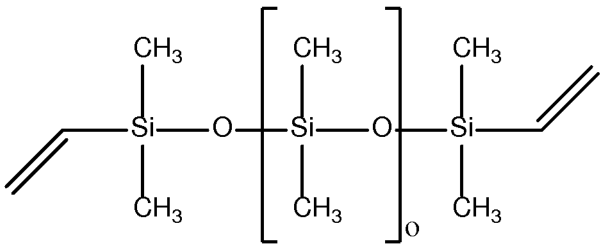

mit O = 5 - 90, bevorzugt 10 - 80 und besonders bevorzugt 15 - 70,
unter Verwendung von
**C** Platin(0)-1,2-divinyl-1,1,3,3-tetramethyldisiloxan, welches durch die Struktur

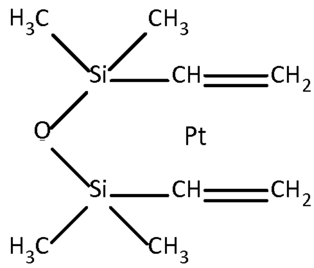

gekennzeichnet ist, als Hydrosilylationskatalysator, durchgeführt wird, wobei das Divinylsilan **B** in einem Silikonöl, vorteilhaft Dimethicon, gelöst und unter Rühren auf Temperaturen im Bereich von 20 - 60 °C, bevorzugt 25 - 45 °C erwärmt wird, und wobei nach Einrühren des Katalysators **C** das Organohydrogensiloxan **A** zugegeben wird.

2. Zubereitungen nach Anspruch 1, **dadurch gekennzeichnet, dass** in dem Silikonelastomer oder den Silikonelastomeren molare Verhältnisse von **A** zu **B** aus dem Bereich von 10:1 bis 1:10, bevorzugt von 5:1 bis 1:5, insbesondere bevorzugt von 2:1 bis 1:2 gewählt werden.

3. Zubereitungen nach Anspruch 1 oder 2, mit einem Gehalt von 0,1 - 60 Gew.-%, bevorzugt 1 - 51 Gew.-%, besonders bevorzugt 3 - 40 Gew.-% an einem oder mehreren Silikonelastomeren nach einem der vorstehenden Ansprüche, bezogen auf die Gesamtzusammensetzung der Zubereitungen

## Claims

1. Cosmetic or dermatological preparations comprising one or more silicone elastomers obtainable or which are obtained by carrying out the reaction of

**A** one or more oligomeric organohydrosiloxanes having at least two SiH-bearing siloxane units per molecule of the following structure

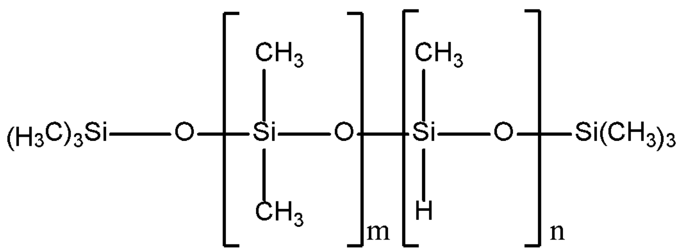

wherein m = 0-40, preferably 0-30, especially preferably 0-20, and
n = 2 - 25, preferably 2 - 15, especially preferably 4 - 10,
and
**B** one or more oligomeric divinylsiloxanes of the following structure

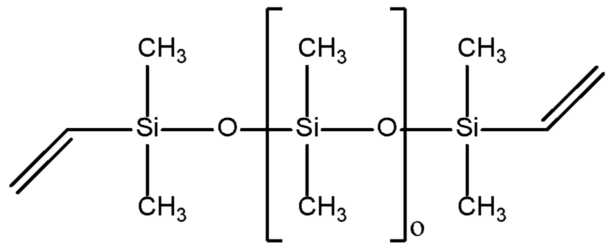

where O = 5 - 90, preferably 10 - 80 and particularly preferably 15 - 70,
with use of
**C** platinum(0)-1,2-divinyl-1,1,3,3-tetramethyldisiloxane which is **characterized by** the structure

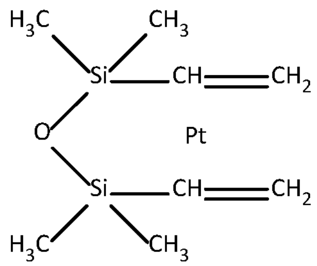

as hydrosilylation catalyst, wherein the divinylsilane **B** is dissolved in a silicone oil, advantageously dimethicone, and is heated with stirring to temperatures in the range of 20 - 60°C, preferably 25 - 45°C, and wherein, after the catalyst **C** has been stirred in, the organohydrosiloxane **A** is added.

2. Preparations according to Claim 1, **characterized in that** in the silicone elastomer(s) molar ratios of **A** to **B** are selected from the range from 10:1 to 1:10, preferably from 5:1 to 1:5, especially preferably from 2:1 to 1:2.

3. Preparations according to Claim 1 or 2 having a content of 0.1-60% by weight, preferably 1 - 51% by weight, particularly preferably 3-40% by weight, of one or more silicone elastomers according to either of the preceding claims, based on the total composition of the preparations.

## Revendications

1. Préparations cosmétiques ou dermatologiques, contenant un ou plusieurs élastomères de silicone, qui peut être obtenu ou qui peuvent être obtenus en ce que la réaction de

A un ou plusieurs organohydrogénosiloxanes oligomèriques comportant au moins deux motifs siloxane portant SiH par molécule de structure suivante

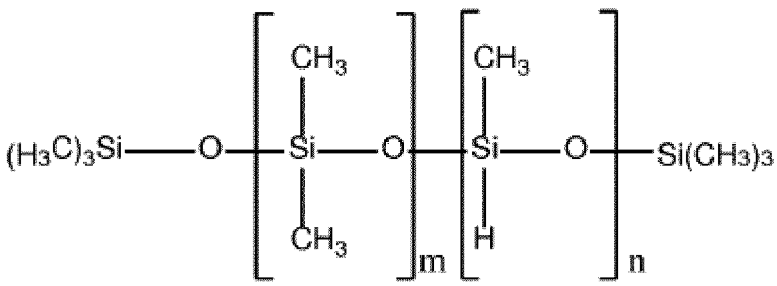

m = 0 à 40, préférablement 0 à 30, en particulier préférablement 0 à 20, et n = 2 à 25, préférablement 2 à 15, en particulier préférablement 4 à 10
et de
B un ou plusieurs divinylsiloxanes oligomériques de structure suivante

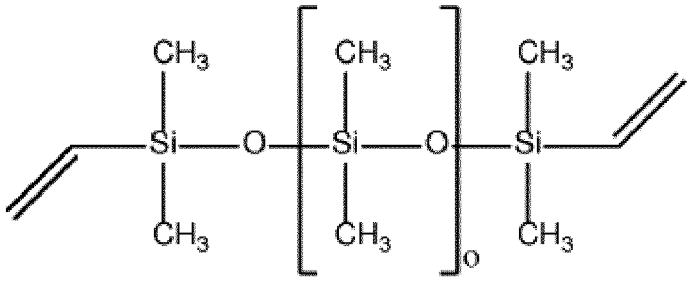

avec O = 5 à 90, préférablement 10 à 80 et particulièrement préférablement 15 à 70, avec l'utilisation de
C 1,2-divinyl-1,1,3,3-tétraméthyldisiloxane-platine(0), qui est **caractérisé par** la structure

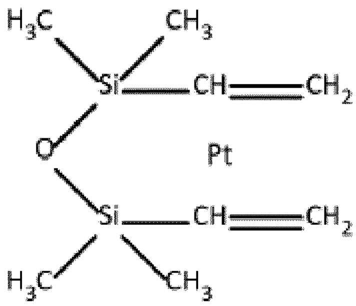

,

en tant que catalyseur d'hydrosilylation, est mise en œuvre, le divinylsilane B étant dissous dans une huile de silicone, de manière avantageuse le diméthicone, et étant chauffé sous agitation à des températures dans la plage de 20 à 60 °C, préférablement 25 à 45 °C, et après délayage du catalyseur C, l'organohydrogénosiloxane A étant ajouté.

2. Préparations selon la revendication 1, **caractérisées en ce que**, dans l'élastomère de silicone ou les élastomères de silicone, des rapports molaires de A à B sont choisis dans la plage de 10:1 à 1:10, préférablement de 5:1 à 1:5, en particulier préférablement de 2:1 à 1:2.

3. Préparations selon la revendication 1 ou 2, dotées d'une teneur de 0,1 à 60 % en poids, préférablement 1 à 51 % en poids, particulièrement préférablement 3 à 40 % en poids d'un ou plusieurs élastomères de silicone selon l'une quelconque des revendications précédentes, par rapport à la composition totale des préparations.

**Fig. 1**

Diffuse Transmission (gemittelt über den Wellenlängenbereich von 400-800 nm).

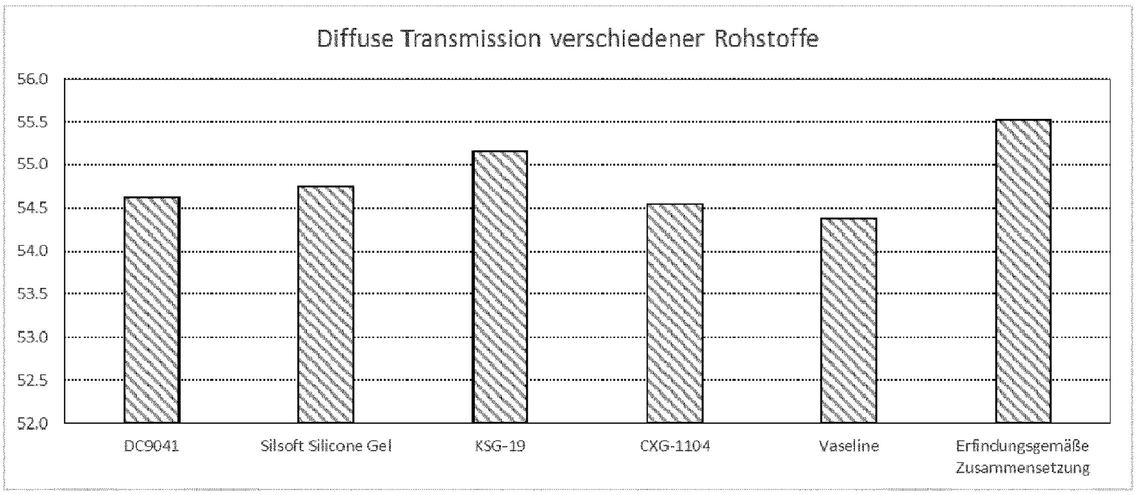

Fig. 2

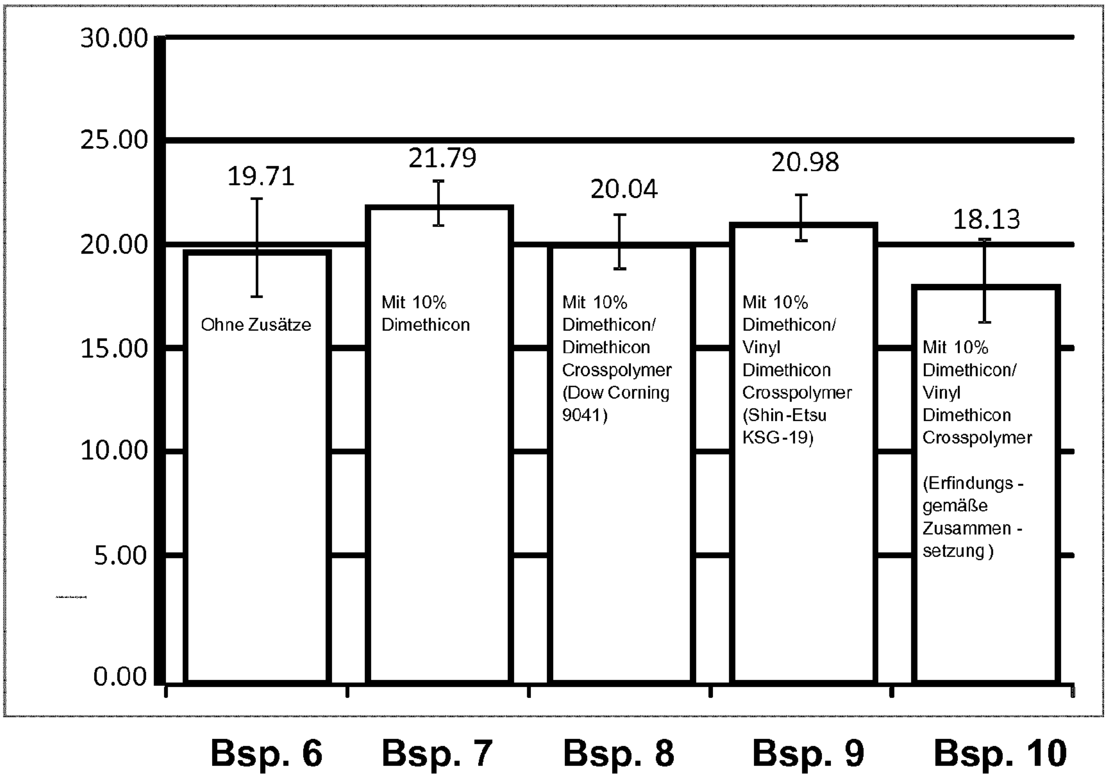
30.00
25.00
20.00
15.00
10.00
5.00
0.00
19.71
21.79
20.04
20.98
18.13
Ohne Zusätze
Mit 10% Dimethicon
Mit 10% Dimethicon/ Dimethicon Crosspolymer (Dow Corning 9041)
Mit 10% Dimethicon/ Vinyl Dimethicon Crosspolymer (Shin-Etsu KSG-19)
Mit 10% Dimethicon/ Vinyl Dimethicon Crosspolymer
(Erfindungs - gemäße Zusammen - setzung)
Bsp. 6
Bsp. 7
Bsp. 8
Bsp. 9
Bsp. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4256870 A
- EP 381166 A
- EP 383540 A
- DE 10155792


### In der Beschreibung aufgeführte Nicht-Patentliteratur


- *Cosm. Toil.,* 1996, vol. 111, 57-61